# EUROPEAN PATENT SPECIFICATION

(11) **EP 4 360 559 B1**
(45) Date of publication and mention of the grant of the patent: **11.06.2025**
(21) Application number: 22383050.6
(22) Date of filing: 31.10.2022
(51) Int. Cl.: A61B 6/03, A61B 6/00

(54) **A TRAINING METHOD OF A COMPUTER-IMPLEMENTED MACHINE LEARNING MODEL FOR OBTAINING A RESTING ENERGY EXPENDITURE (REE) PARAMETER OF A SUBJECT**
TRAININGSVERFAHREN EINES COMPUTERIMPLEMENTIERTEN MASCHINENLERNMODELLS ZUM ERHALT EINES RUHEENERGIEVERBRAUCHSPARAMETERS EINER PERSON
PROCÉDÉ D'APPRENTISSAGE D'UN MODÈLE D'APPRENTISSAGE AUTOMATIQUE MIS EN UVRE PAR ORDINATEUR POUR OBTENIR UN PARAMÈTRE DE DÉPENSE ÉNERGÉTIQUE AU REPOS (REE) D'UN SUJET

(43) Date of publication of application: 01.05.2024
(73) Proprietor: Fundació Hospital Universitari Vall d'Hebron - Institut de Recerca, 08035 Barcelona (ES); Artis Tech Development, S.L., 35017 Las Palmas de Gran Canaria (ES)
(72) Inventor: Simó Canonge, Rafael, 08173 SANT CUGAT DEL VALLÈS Spain (ES); Palmas Candía, Fiorella Ximena, 08035 BARCELONA (ES); Ciudin Mihai, Andreea, 08035 BARCELONA (ES); Guerra Hernández, Raúl Celestino, 35400 ARUCAS (ES); Melián Álamo, José María, 35019 LAS PALMAS DE GRAN CANARIA (ES)
(74) Representative: ZBM Patents - Zea, Barlocci & Markvardsen

(56) References cited:
- CAROLINE NERVIL KISTORP ET AL: "Measurements of Body Composition by Dual-Energy X-ray Absorptiometry Improve Prediction of Energy Expenditure", ANNALS OF THE NEW YORK ACADEMY OF SCIENCES, NEW YORK ACADEMY OF SCIENCES, US, vol. 904, no. 1, 25 January 2006 (2006-01-25), pages 79 - 84, XP071393066, ISSN: 0077-8923, DOI: 10.1111/J.1749-6632.2000.TB06424.X
- DABIRI SETAREH ET AL: "Deep learning method for localization and segmentation of abdominal CT", COMPUTERIZED MEDICAL IMAGING AND GRAPHICS, PERGAMON PRESS, NEW YORK, NY, US, vol. 85, 14 August 2020 (2020-08-14), XP086323539, ISSN: 0895-6111, [retrieved on 20200814], DOI: 10.1016/J.COMPMEDIMAG.2020.101776
- HEYMSFIELD STEVEN B ET AL: "The anatomy of resting energy expenditure: body composition mechanisms", EUROPEAN JOURNAL OF CLINICAL NUTRITION, NATURE PUBLISHING GROUP, GB, vol. 73, no. 2, 25 September 2018 (2018-09-25), pages 166 - 171, XP036861376, ISSN: 0954-3007, [retrieved on 20180925], DOI: 10.1038/S41430-018-0319-3
- CUNNINGHAM J J: "BODY COMPOSITION AS A DETERMINANT OF ENERGY EXPENDITURE: A SYNTHETIC REVIEW AND A PROPOSED GENERAL PREDICTION EQUATION", AMERICAN JOURNAL OF CLINICAL NUTRITION,, vol. 54, no. 6, 1 December 1991 (1991-12-01), pages 963 - 969, XP009044196, ISSN: 0002-9165

## Description

The present disclosure relates to methods and systems used for the estimation of Resting Energy Expenditure (REE) parameters of a subject.

### BACKGROUND

Recent research has focused on the development of new techniques able to evaluate the human body composition. A first example is the technique known as Magnetic resonance imaging (MRI), which is a type of scan that uses strong magnetic fields and radio waves to produce detailed images of the inside of the body. Another example is Computed tomography, commonly referred to as CT scan, which is able to precisely measure the attenuation of X-ray passing through different types of tissues (i.e., fat, muscle, etc.). However, in contrast to the high amount of technology involved in the acquisition of data when using such techniques, there is a clear lack of tools that allow an efficient and precise analysis of the acquired data. For instance, in most cases, the information within MRI scans or CT scans is manually evaluated by a physician or trained technician performing a visual inspection. Consequently, this leads to a lack of precise quantitative measurements, repetitive and time-consuming tasks, as well as inter-observer dependencies in diagnostics and even possible human errors.

In order to improve measurements of MRI or CT scans, software tools have emerged aimed at performing quantitative analysis of human body composition, which can be useful for specific purposes in daily clinical practices and/or research activities. These tools mostly provide quantitative information about the volume or area of different tissues and usually require manual selection of the tissues to be quantified, based on the images from MRI or CT scan.

Different approaches for the estimation of the REE may be based on the use of imaging techniques, such as MRI and CT scans, which allows to obtain an organ and/or tissue volumes. Such methods may include, knowing the average density of each target organ/tissue, estimating the mass of a target organ/tissue, for example in kilograms, as its volume multiplied by its average density. Organ-tissue metabolic rates (OMR), measured in kJ/(kg·day), have been proposed in earlier literature. Each individual organ-tissue mass is then multiplied by its corresponding OMR obtaining the REE for that specific organ-tissue. The calculation of the whole body REE (kJ/day) of a subject is obtained as the sum of the REE for every individual organ/tissue. The mentioned procedure may also be applicable to other imaging methods like dual-energy X-ray absorptiometry (DXA), whose main limitation is that tissue quality or density cannot be estimated. Furthermore, the literature assumes that different organs and tissues are homogeneous in composition. Therefore, subjects with diseases as muscular dystrophy (i.e., lipid replacement of skeletal muscle fibers) or hepatic steatosis (i.e., fatty infiltration of liver) might obtain an erroneous or low accuracy-REE estimation. Additionally, the state-of-the-art methods to obtain the REE may require mostly whole-body information, which may imply a high quantity of radiation to the subject when CT scans are involved. Document "Measurements of Body Composition by Dual-Energy X-ray Absorptiometry Improve Prediction of Energy Expenditure" by Caroline Nervil Kistorp Et Al discloses a method of assessing body composition by performing a classical statistical linear regression analysis between body composition in terms of fat tissue and lean tissue, obtained from DXA scans, with actual REE values.

REE estimations in the state of the art based on imaging techniques have a low accuracy and can be affected by factors related to a specific subject.

### SUMMARY

The present disclosure aims at defining a method for an accurate estimation of a REE parameter of a subject based on imaging techniques, particularly characterized in that the REE parameter is obtained based on information extracted from, at least, one image of a computed tomography CT scan of a subject.

In a first aspect, a training method of a computer-implemented machine learning model for obtaining a Resting Energy Expenditure (REE) parameter of a subject is provided, as defined in the appended claimsThe method comprises the following steps:
obtaining one or more ground-truth REE parameters of one or more subjects; the estimation may have been performed using any measurement methodology, for example, by an indirect calorimetry (IC) measurement of a subject or by bioimpedanciometry;
obtaining the one or more CT scan images of the one or more subjects; the CT scan image(s) may be centered at any level of the subject: in some examples the CT scan image may be centered at leg level, and/or abdomen level, thorax, neck, etc. There are references in the literature of the estimation of body composition in all of them, the most accurate being the abdominal L3 level; each ground-truth REE parameter is associated with each subject and therefore with each CT scan image;
identifying at least a region of interest, ROI, in each of the obtained CT scan images. This ROI may correspond to one specific tissue (such as muscle, visceral adipose tissue, etc.), to one specific organ (for example, liver, kidney, brain, etc.), or any combination of them, including the whole body of the subject within the CT scan image(s);
calculating a group of parameters of each ROI; wherein a group comprises one or more parameters of each ROI, and wherein each group is associated with a ground-truth REE parameter. The parameter comprises a radiodensity of at least one ROI. The parameter may comprise a quantity expressed in area of the ROI, volume of the ROI, percentage, etc. Each calculated group of parameters of a CT scan image of a particular subject is associated with a ground-truth REE parameter of the particular subject;
performing a supervised training of the computer-implemented machine learning model, using a training data set comprising one or more groups of parameters of each ROI and the associated one or more ground-truth REE parameters, and wherein the training further comprises, for each group, setting an output parameter of the model corresponding to the associated ground-truth REE parameter.

Methods in the state-of-the-art show significant correlation between the quantity of a tissue, such as, for example, muscle mass, measured in a CT scan image of a human body, and the measurement of the total quantity of the same tissue within the whole body, for example, the total amount of body muscle mass. More precisely, it is also possible to draw such correlation in the case of other tissues of the body, such as, for example, visceral adipose tissue (VAT) or subcutaneous adipose tissue (SAT), the correlation being between the quantity of tissue measured in a CT scan image, and the total amount of said tissue within the whole body. Methods in the state-of-the-art may suggest that the value of the Resting Energy Expenditure (REE) measurement i.e., the energy expenditure while a person is at rest, awake, and in an interprandial state, of the same human body may strongly depend on the total amount of fat free mass.

The methods defined in the present disclosure allow estimating or inferring or calculating REE parameter departing from a CT scan image of a subject. The methods defined in the present disclosure allow correlating information obtained from, at least, one image with a Resting Energy Expenditure (REE) parameter in a scalable or reproducible manner.

The REE parameter can be estimated for all type of subjects, healthy subjects or patients. Among the subjects, different objectives may be pursued, for example, the knowledge of REE parameter may be used for adapting a diet or exercise for a healthy subject or a patient.

The methods of the present disclosure may be implemented for a baseline evaluation, and for follow-up, to evaluate, for example, evolution in time, including for example physiological aging, to see how body composition and REE change with age or in various stages and circumstances of life, response to treatments, response to diets, response to exercise or response to stress.

A subject may undergo an indirect calorimetry (IC) test to obtain a ground-truth REE parameter from the subject. Other measurement/estimation techniques may be employed, such as bioimpedanciometry. An IC test (the actual gold-standard method for REE measurements) may calculate heat that living organisms produce by measuring either their production of carbon dioxide and nitrogen waste (frequently ammonia in aquatic organisms, or urea in terrestrial ones), or their consumption of oxygen. This technique provides unique information, is noninvasive, and can be advantageously combined with other experimental methods to investigate numerous aspects of nutrient assimilation, thermogenesis, the energetics of physical exercise, and the pathogenesis of metabolic diseases.

Generally, in order to perform an IC test, a machine may be used which captures the gases that the subject breathes in and out, in order to measure a parameter corresponding to the subject's REE. Therefore, IC tests are used as reference tests (gold-standard) to measure how much energy the subject expends at rest (in other words, how many calories the subject is burning while resting). Even though IC is an accurate measurement of the subject's REE, it is cumbersome to perform, the machinery used to perform it is expensive, and it needs specially conditioned space and trained personnel for its operation. Therefore, it is rarely performed on the subject in the daily practice setting.

In parallel, or either before or after the ground-truth REE measurement technique is performed, preferably, for example, within no more than one week, a CT scan may be performed on the subject, and a set of CT scan images may be obtained (comprising at least one image).

A CT scan image may comprise the following information when obtained:
- the area and/or volume corresponding to a region of interest (ROI) can be measured based on the number of pixels of the CT scan image corresponding to that ROI, and the pixel resolution (measured, for example, in cm²/pixel or cm³/pixel).
- Information about the region of interest (ROI). Generally, each pixel of a digital CT scan image represents the attenuation of the X-rays which pass through the tissue of the body. Therefore, the specific attenuation may be proportional to the density of the tissue; the radiodensity is commonly measured in Hounsfield Unit (HU). Therefore, a parameter related to the radiodensity or density of the ROI can be obtained, such as, for example, the average HU.

The CT scan image to be used may be located at different positions of the human body, such as, for example, subject's leg, abdomen, thorax, neck, etc. Depending on that location, more or less relevant data may be obtained to estimate the REE.

For example, at L3 vertebral level, the psoas muscle is very wide, and therefore useful information may be retrieved from a CT scan image performed at this level. Furthermore, at such level which corresponds to an abdomen level, other more prominent tissues are found, such as: visceral adipose tissue (VAT) (excess fat, which tends to accumulate between the intraabdominal organs or inside them), or subcutaneous adipose tissue (fat which accumulates under the skin).

Even though any other location may be used to perform the scan, L3 vertebral level is a location which statistically correlates best the amounts of fat and muscles tissues and its features within the body as a whole.

Other parts of the body or other abdominal vertebral levels, such as L5 or L7, may not correlate the muscle and fat tissues as exactly as in L3, and thus other parts may not be as representative of the body as a whole (regarding fat and muscle) but would still be useful to perform an REE estimation. Regardless of the chosen part of the body, the training procedure of the present disclosure may not vary.

Once one or more CT scan images are obtained, at least a region of interest, ROI, is identified and at least one parameter of the ROI is calculated, for example, an area, or a volume or a density. The ROI may correspond to a subject's area or volume that may include, at least, one tissue (i.e., muscle, visceral adipose tissue, subcutaneous adipose tissue, etc.), one organ (i.e., liver, kidney, brain, heart, etc.) or any combination of them, including an entire subject's body within the image. Derived information, such as size (i.e., cm², cm³, etc.), radiodensity (in Hounsfield Unit, HU) or any other metric can be obtained from that ROI. In the present disclosure "entire subject's body within the image" may be understood as a region of a CT scan image representing or showing at least a part of a subject's body. For example, entire subject's body within the image may comprise a slice of a body abdomen resulting from a transversal cut and representing the whole/entire perimeter of the abdomen, with the tissues and organs included. For example, entire subject's body within the image may comprise a slice of a leg resulting from a transversal cut and representing the whole/entire perimeter of the leg, with the tissues and bones included. For example, entire subject's body within the image may comprise a slice of a part of a body resulting from a transversal cut and representing a whole/entire at least a region of the part of the body, not necessarily the whole or entire perimeter.

In some examples, the ROI may represent a specific tissue or organ in the image. Identifying a ROI may comprise performing a segmentation of the one or more obtained images. At least one predetermined type of tissue or organ, or any combination of them, including the entire subject's body within the image may be identified. More precisely, in segmentation, different tissues or organs can be identified on the image, such as, for example, muscle, subcutaneous adipose tissue, visceral adipose tissue or intramuscular adipose tissue. Such segmentation may be performed by a computing system, by identifying different types of tissues within the CT scan image or may be performed by receiving data related to such areas of the CT scan image, from, for example, a Graphic User Interface (GUI), wherein a physician or a trained technician has visually identified and marked parts of the CT scan image and has inputted such identified parts into a computing system by inputting means, for example a mouse, a keyboard, or a touchpad.

After a ROI is identified, at least one ROI parameter may be calculated related to an amount of the identified ROI, from the segmented image, wherein an amount may comprise an area or a volume.

An example of a ROI parameter may be a premeasured quantity, for example, an area or volume in cm² or cm³. In such example, the pixels of the CT scan image occupied by a ROI may be counted, and a conversion from pixels to, for example, cm² may be performed, using the pixel resolution provided within metadata which may be integrated in the CT scan image.

Alternatively, a parameter related to the percentage of a ROI with respect to the total subject's body within the image may also be calculated. This percentage represents a relative measure of a specific ROI within the subject, thus giving a more representative measurement of the amount of ROI within a person's body. For example, two subjects may have a similar amount of muscle, but one of them may be fatter than the other. Thus, in the thinner subject, the amount of muscle may be the same as in the fatter subject, but the relative amount of muscle may be higher, thus resulting in different energy expenditure. Therefore, using a percentage may lead to a more accurate estimation of REE. Furthermore, different measurements (i.e., parameters) may be used for different identified ROIs, in order to use a combination which may enhance the accuracy of REE estimations. Different groups combining different types of ROIs segmented from a CT scan can be obtained and subsequently used as a training data set, to infer a more accurate REE parameter of the subject. More precisely, the resulting REE estimation may consider different scenarios, wherein different types of tissues have different energy expenditures, and the same type of tissue may have a different energy expenditure depending on the ratios of tissue in each body and the type of body itself.

Finally, the supervised training of a machine learning model is performed, using a training data set which comprises one or more groups of the calculated parameters corresponding to one or more subjects. More precisely, the supervised training may be performed using a training data set wherein the data is organized at least in pairs. The pairs may comprise a group that includes at least one calculated parameter of the ROI of a CT scan image of a subject, and an output parameter corresponding to the ground-truth REE parameter measured on the same subject, for example by means of an IC test or bioimpedanciometry. The received training data set may be processed through the machine learning model. The machine learning model may implement supervised learning logic, wherein the model is given the training data set to ingest and told a result to be interpreted from the data -supervised-. The result is the ground-truth REE parameter measured on the subject for each respective obtained CT scan image. The model is trained to arrive at the result based on the ingested data and the accuracy of the trained model is dependent in part on the diversity of the received training data set being ingested. A trained model results from the supervised training. The trained model may result when a deviation or error or validation error between the output of the model and a reference/real/ground-truth REE parameter is lower than, for example, 5% or lower than a 10%. The deviation may be tested during a validation of the model.

The training data set may further include, in some examples, numerical variables or values such as anthropometric information of subjects, height, weight, age, information from the CT image, areas or volumes of different ROIs measured, for example, in cm², cm³ and/or in % and averages of Hounsfield units, among others. The training data set may also include categorical variables such as a subject's gender, demographic information, race, clinical information, etc. Such variables may be obtained by the disclosed methods by receiving them from an external source or by calculating them. When a system implements the methods disclosed herein, the system may comprise reception means, such as an interface, for receiving the input variables and CT scan image, or calculating means such as a processor, for calculating one or more of the variables used for training the model. When calculating the variables, the method disclosed herein may calculate one or more of them from the CT scan image.

By using the previously described method, a machine learning model may be trained in a way in which, when provided with processed data extracted from a CT scan image of a subject, the machine learning model provides a REE parameter which may be more accurate than an REE parameter provided by other REE estimation methods.

In some examples, the steps of obtaining one or more ground-truth REE parameters of one or more subjects; obtaining the one or more CT scan images of the one or more subjects; identifying at least a ROI; and calculating one or more parameters of each ROI may be performed by a first entity or processor. The parameters may then be sent by means of a wired or wireless connection to a second entity or second server which may be configured to perform the step of performing a supervised training of the computer-implemented machine learning model, using a training data set comprising the calculated one or more parameters of each ROI and associated ground-truth REE parameters, and wherein the training further comprises for each of the one or more parameters of each ROI, setting an output parameter of the model corresponding to the associated ground-truth REE parameter.

Generally, a CT scan is performed more regularly and is much easier to perform than a method for obtaining an REE parameter experimentally, such as an IC test or bioimpedancemetry, which is physically demanding on the subject, involving cumbersome and error-sensitive equipment. Therefore, by performing the above-described method, once the model is trained, the model may be stored in non-volatile memory or non-transitory computer readable media to be used for estimating a further REE parameter.

Methods for obtaining an REE parameter using a trained model as described herein avoids experimental measurement methods for determining REE parameters of new subjects. More precisely, by using a machine learning model trained by the above-described method steps, a REE parameter may be obtained by inputting information extracted from one or more CT scan images to a system where the trained machine learning model runs and obtaining, from the model, the REE parameter. The methods of the present disclosure avoid obtaining an REE parameter experimentally (i.e., by IC tests or by bioimpedanciometry), while obtaining an REE parameter with a similar accuracy to the REE estimation method used to provide the training data set.

While literature describes methods for estimating a REE parameter departing from CT images, the method proposed herein provides a more accurate correlation between CT scan images and real or ground-truth REE measurements. Furthermore, the methods described herein may be more efficient and less computationally demanding, since the REE parameter can be inferred using information obtained from even one single CT scan image.

In examples, the computer-implemented machine learning model may be a regression model since the expected result is an estimated numerical value (corresponding to the ground-truth REE of the subject). Different types of machine learning models may be used. The computer-implemented machine learning model may be one of a neural network, or a model comprising assembled decision trees, or a neural network comprising at least one fully connected layer, or a combination of one or more neural networks and one or more decision trees used to execute regression.

For example, the machine learning model may be an artificial neural network ANN. ANNs have a very high performance when the datasets to be used both for training and, subsequently, for regression, are composed of homogeneous data. It may be understood that the data in a dataset are homogeneous when they are made up only of elements of the same type, such as, for example, numerical data, whether discrete or continuous. These datasets may include only numerical variables, containing information of the subjects, such as: anthropometric information (height, weight), demographic information (age), information from the CT scan (quantity of different ROIs in, for example, cm², cm³ and/or % and radiodensity values). ANNs are very efficient at modelling non-linear relationships between multiple variables. They are also very useful for finding relationships between variables that are difficult to predict in a first instance.

During the training process of the ANN, weights of the ANN are calculated which assign greater or lesser importance to each of the existing relationships between the different input parameters inputted in the network. The number of trainable parameters of the network, i.e., weights and bias, directly depends on the number of layers and the number of neurons per layer and the type of layers used.

One of the criteria by which ANNs can be classified is based on the degree of connection between their neurons, a classification that divides networks into partially or fully connected. Fully connected networks are those whose layers are all of the fully connected type. All the neurons in a fully-connected ANN in a layer are connected to all the outputs of the neurons of a previous layer. Among all the possible architectures of NNs, those including fully- connected layers (also known as densely-connected) may be very suitable to perform regression. As an example, the fact that the model used may comprise at least one fully connected layer allows
- during the training process, each of the at least one fully connected layer automatically finds all the existing relationships between the outputs of the neurons of a previous layer;
- during a neural network design stage, the fully connected layer can be used to modify the dimensions of the network, i.e., it is possible to vary the number of neurons between successive layers. This is very useful when the fully connected layer is used as the output layer of the neural network as it allows adjusting the number of estimations to be performed. During tests for testing the methods and systems of the present disclosure, this fact has allowed to go from N variables in the input layer of the network to a single output corresponding to the estimation of the basal energy expenditure (REE).

The machine learning model may be also, for example, based on "decision trees". This kind of machine learning models is especially useful when the set of input parameters is heterogeneous. Heterogeneous sets of input parameters may contain any combination of different types of parameters, such as numerical variables (i.e., area/volume, radiodensity, height, weight, age, etc.) and categorical ones (i.e., gender, race, clinical information, etc.). Decision trees are machine learning models that aim to build a model that can predict the value of a target variable. These may be represented with a tree structure, that uses branches to illustrate the output of each decision. Each node represents a test on a specific variable and each branch represents the result of that test. These models use simple decision rules that infer the expected outcome, known as leaves, from the characteristics of the input data.

However, the use of a single decision tree tends to produce overfitting on the training data. The use of such a model to infer a result with real data can therefore produce a high error. Ensembled decision trees models may be used to profit from the benefits of decision trees when working with heterogeneous data, while reducing overfitting. In this way, the predictions of a multitude of decision trees may be combined to generalize the machine learning model and make it robust. This allows the variance of the results of the individual decision trees to be reduced. Therefore, a category of machine learning models which may work for the methods and systems of the present disclosure may be based on ensemble decision trees, i.e., learning algorithms that contain at least two decision trees. There are currently at least two families of learning models based on ensemble decision trees:
- Averaging methods. Among others, there are Bagging and Random Forest methods. These methods are constructed with several independent trees and provide a result that is the average value of the predictions of each decision tree separately. This results in the reduction of the variance of the predictions of each independent decision tree.
- Boosting methods. Algorithms such as AdaBoost, Gradient Boosting Decision Tree and Light GBM, among others, belong to this category. With this methodology, decision trees are incorporated sequentially, so that a decision tree tries to reduce the error produced by its predecessors. In this way, the combination of several weak models produces a robust, efficient, and accurate one.

For using any machine learning model for regression, such as those previously described, to estimate the REE parameter, a supervised training strategy may previously be performed to train the model.

As an example, a cross-validation may be employed when training the machine learning model for regression to prevent overfitting or underfitting. As an example, a stratified k-fold cross validation strategy may be followed. This strategy consists in separating the data set in k subsets arrange in such a way that each subset is a good representation of the entire data set. Then, k-1 subsets are grouped as the training set, and the other subset is the testing set. In this way, the training is executed using k-1 subsets (training set), and the model is validated using the remaining subset (testing set). This is repeated k times, leaving out a different subset each time (as testing set), Finally, the average validation error is obtained as the average of the validation error obtained in each of the k iterations for the testing set. Similarly, the average training error is obtained by averaging the error in the training set in the k iterations. By doing so, overfitting may be identified when the validation error is considerably higher than the training error. On the contrary, underfitting is identified when both the training error and the validation error are high. If the machine learning model consists in an ANN, additional techniques may be also used to prevent overfitting. For example, dropout regularization may be used. If the machine learning model is based on decision trees, additional techniques may be also used to prevent overfitting. For example, pruning strategies, such as bottom-up pruning or top-down pruning may be used.

As another example, to carry out a good selection of the hyperparameters involved in the machine learning model for regression, hyperparameters tuning may be executed during the training. This may also reduce the overfitting. As an example, the hyperparameter tuning may be executed based on a grid search strategy, where all the possible combinations of the hyperparameters are exhaustively tested and the combination that provides the best results is preserved. As another example, the hyperparameter tuning may be also executed based on a randomized search or a Bayesian optimization to reduce the number of combinations of hyperparameters to be tested and to reduce the overall training time.

A supervised training strategy to train the model may also be based on the following. In examples, the training data set may comprise data from different subjects with different ages, types of bodies, different ROIs and different parameters associated to these ROIs, or even health problems. Using such training data sets with information from different subjects, the REE parameter is estimated based on all the input parameters. Including different types of subjects in the training data set may result in a more accurate regression model for estimating the REE for any kind of subject.

Preferably, in an example, in order to obtain a more precise estimation, both the CT scan image and the obtaining of the ground-truth REE parameter using experimental test (i.e., indirect Calorimetry (IC) or bioimpedancemetry) may be performed within a relatively short period of time between them (for example, within one week). Within such timeframe, the characteristics of the subject, such as, for example, its body composition, its clinical condition, etc. may not vary significantly, and the resulting REE parameter of the subject may be more accurate.

In examples, a parameter referring to the quantity of, at least, a specific ROI may be calculated in different ways. One of them may be to count the number of pixels in a CT image corresponding to that specific ROI and multiply it by the pixel resolution (for example in cm²/pixel or cm³/pixel), thus obtaining the area (in cm²) or the volume (in cm³) corresponding to that ROI. Other way may be to divide the number of pixels corresponding to that ROI by the number of pixels corresponding to other ROI, thus obtaining a relative measurement that can be represented, for example, in percentage (%).

In another example, a parameter referring to the radiodensity of, at least, a specific ROI may be calculated in different ways. One way may be to compute the average value of the radiodensity of that specific ROI. This may be done by averaging the radiodensity value of all the pixels included in that specific ROI, measured, for example, in Hounsfield Units (HU). The radiodensity, in HU, corresponding to each image pixel may be included in the CT scan metadata, or at least, computed based on the CT scan metadata.

According to a further example, parameters over the plurality of CT scan images may be combined for a single subject. For example, two CT images captured at different vertebral levels may be used. For example, at least one ROI may be identified in each CT scan image corresponding, for example, to a specific tissue such as muscle tissue or adipose tissue. Furthermore, a parameter may be obtained for the target ROI as the average value of that parameter obtained for that target ROI in both images. While this kind of strategy may increase the estimation accuracy of the REE parameter, it also increases the computational cost.

In case of using software tools, including semiautomatic labeling of scans, when performing the segmentation or ROI identification, the overall training method may be streamlined and it may further allow the machine learning model to keep improving its performance in future analysis of new CT scans (i.e., when inferring the REE parameter with information from new scan images).

Additionally, other subject data can also be added as input parameters of the machine learning model, to increase the model accuracy. Such data may comprise, for example, demographic data, anthropometric data. Furthermore, REE measurements may also change according to the subject clinical state. Accordingly, clinical information regarding the subject state may also be added to increase the model accuracy.

According to an example of the present disclosure, a method for estimating a Resting Energy Expenditure (REE) parameter of a subject is also provided, comprising the steps of:
- obtaining at least one computed tomography CT scan image of the subject;
- identifying at least a region of interest, ROI, of the CT scan image;
- calculating at least one parameter of each ROI;
- inferring or estimating the REE parameter using a supervised machine learning model trained using the training method of a computer-implemented machine learning model previously described, wherein the inferring comprises inputting a data set comprising the at least one calculated ROI parameter, to obtain an REE parameter as the output of the model.

In some examples the steps of obtaining at least one computed tomography CT scan image of the subject; identifying at least a region of interest, ROI, of the CT scan image; and calculating at least one parameter of each ROI are performed by a first entity and the inferring or estimating the REE parameter is performed by a second entity. An entity may be understood as a processor, or processing means or computer.

In another aspect, a computer program product is defined in the appended claims. The computer program product may comprise program instructions for causing a computing system to perform a supervised training of the computer-implemented machine learning model, using a training data set comprising one or more groups of parameters of respective ROI of CT scan images and associated one or more ground-truth REE parameters, and wherein the training further comprises, for each group, setting an output parameter of the model corresponding to the associated ground-truth REE parameter.

The computer program may further comprise program instructions for causing the computing system to
- obtain one or more ground-truth REE parameters of one or more subjects;
- obtain the one or more CT scan images of the one or more subjects;
- identify at least a region of interest, ROI, in the obtained CT scan images;
- calculating a group of one or more parameters of each ROI.

The computer program product may be embodied on a storage medium (for example, a CD-ROM, a DVD, a USB drive, on a computer memory or on a read-only memory) or carried on a carrier signal (for example, on an electrical or optical carrier signal).

The computer program may be in the form of source code, object code, a code intermediate source and object code such as in partially compiled form, or in any other form suitable for use in the implementation of the processes. The carrier may be any entity or device capable of carrying the computer program.

For example, the carrier may comprise a storage medium, such as a ROM, for example a CD ROM or a semiconductor ROM, or a magnetic recording medium, for example a hard disk. Further, the carrier may be a transmissible carrier such as an electrical or optical signal, which may be conveyed via electrical or optical cable or by radio or other means.

When the computer program is embodied in a signal that may be conveyed directly by a cable or other device or means, the carrier may be constituted by such cable or other device or means.

Alternatively, the carrier may be an integrated circuit in which the computer program is embedded, the integrated circuit being adapted for performing, or for use in the performance of, the relevant methods.

### BRIEF DESCRIPTION OF THE DRAWINGS

Non-limiting examples of the present disclosure will be described in the following, with reference to the appended drawings, in which:
Figures 1 and 2 depict two different examples of a computer-implemented neural network for obtaining a Resting Energy Expenditure (REE) parameter of a subject, according to the present disclosure.
Figure 3 depicts an example of a CT scan image used by a method according to the present disclosure.
Figure 4 depicts a flow chart of an example of the training method of any of the neural networks depicted in figures 1 and 2, according to the present disclosure.
Figure 5 depicts a solution according to the present disclosure.
Figure 6 depicts a system for providing an estimated REE parameter of a subject.
Figure 7 depicts a system for providing an estimated REE parameter of a subject.

### DETAILED DESCRIPTION OF EXAMPLES

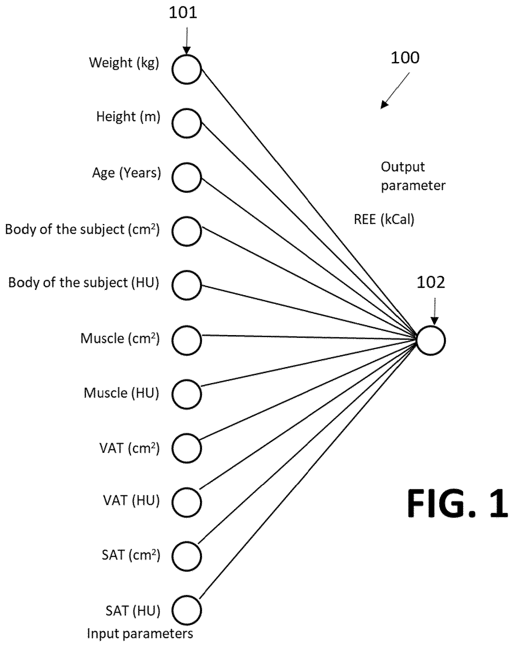

Figure 1 depicts a neural network which may be trained by one of the training methods according to the present disclosure. Figure 1 depicts a machine learning model embodied in a fully connected neural network 100. In this specific embodiment, the machine learning model is a neural network comprising only fully connected layers 101 and 102 resulting in a fully connected neural network 100. In this case, the neural network comprises only the input and output layer and does not include any hidden layer.

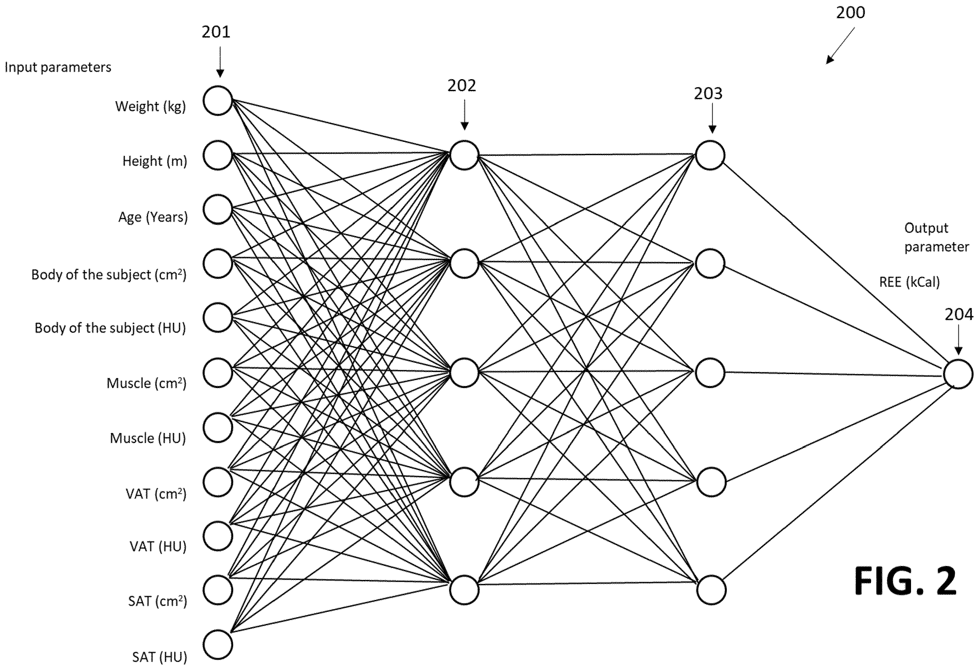

Figure 2 depicts a machine learning model embodied in a fully connected neural network 200. In this specific embodiment, the machine learning model is a neural network comprising only fully connected layers 201, 202, 203 and 204 resulting in a fully connected neural network 200. In this case, the neural network comprises two hidden layers 202 and 203.

In the present example, as seen in figure 2, the number of neurons in the input layer 201 and output layer 204 of neural network 200 corresponds to the number of input and output parameters, respectively. In this case, the number of input parameters is eleven: Weight, Height and Age of the subject, area of subject's body within the image (in cm²), radiodensity of subject's body within the image (in HU), area of Muscle (in % related to subject's body within the image), radiodensity of Muscle (in HU), area of VAT (in % related to subject's body within the image), radiodensity of VAT (in HU), area of SAT (in % related to subject's body within the image), and radiodensity of SAT (in HU). As seen, the methods of the present disclosure include that the one or more parameters of each ROI comprise a percentage of an identified ROI, relative to a tissue or an organ or to any combination of thereof, with regards to a different ROI relative to other tissues, other organs, or any combination of thereof, including the entire subject's body within the obtained CT scan image.

As seen, in a neural network comprising at least one fully connected layer, each neuron in a fully connected layer is connected to all neurons in the previous layer. In this way the resulting neural network may consider multiple relationships between the input parameters to achieve an output value. The methods and systems according to the present disclosure may use different and varied physical features of the subjects to construct a training data set; many different relationships may therefore be established between the input parameters of the neural network. For example, a body with a high amount of muscle may commonly mean that the subject is fit, but if a further input parameter related to the radiodensity of the muscle tissue shows a low radiodensity value it may suggest that the patient is not as fit, and it may indicate a lower REE. As another example, a body with a high amount of muscle may commonly mean that the subject is fit, but if another input parameter indicating a high amount of visceral adipose tissue, it may suggest that the subject is not so healthy, which may affect his REE. Therefore, hidden relationships between input parameters may be considered when using a neural network comprising at least one fully connected layer, and its training may lead to more accurate REE parameters in further inferences for new subjects.

In the examples of figure 1 and figure 2, the CT scan image may be obtained at L3 vertebral level of a subject, in order to obtain the maximum correlation of an area or volume of tissues found in the CT scan image in L3, and the amount of the same tissues in the whole body of the subject.

In another example, if the amount of data available for training is considerably large (in the order of thousands of samples), the complexity of the model may be increased, and other models may be used, including other types of neural networks, or decision trees, any combination thereof, etc.

As an example, if the amount of data available for training is considerably large (in the order of thousands of samples), an ANN with a larger number of input parameters and a larger number of trainable parameters than the examples of figures 1 and figure 2 may be used. By increasing the input parameters and trainable parameters (the total amount of weighs and biases of the ANN as a whole), a more accurate estimation of a REE parameter may be obtained.

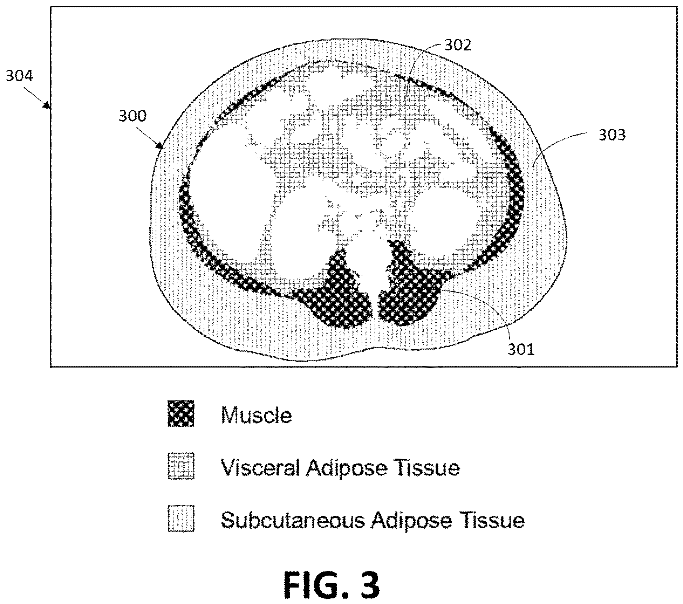

Figure 3 depicts an example of a CT scan image 304 of vertebral level L3 used by a method of the present disclosure. In this figure, the entire subject's body within the obtained CT scan image may be the region referenced as 300. The image is represented as segmented, wherein different tissues are differentiated as follows: muscle 301, visceral adipose tissue 302, subcutaneous adipose tissue 303.

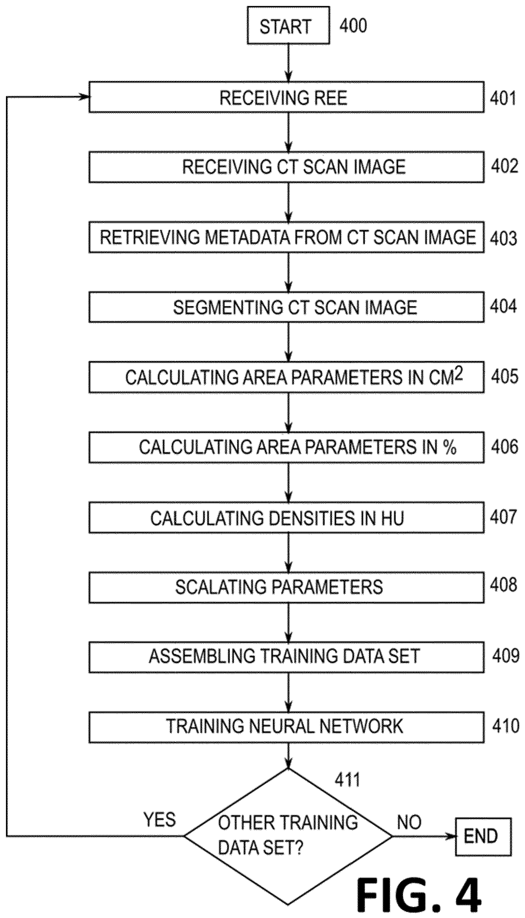

Figure 4 depicts a flow chart of an example of the training method of any of the neural networks depicted in figure 1 or figure 2, for obtaining a Resting Energy Expenditure (REE) parameter of a subject according to the present disclosure. In the present example, the method is performed by a computing device which comprises a fully connected untrained neural network.

More precisely, in step 401 of figure 4, the method starts by the computing device receiving a ground-truth REE parameter obtained from an indirect calorimetry measurement or by bioimpedanciometry of a subject.

In step 402, the computing device receives an axial computed tomography CT scan image of the same subject. The image may be centered at L3, or L5 or L7 vertebral level or at a leg level or at thorax level or at neck level, or any other level. The CT scan image is embedded in a digital file, comprising the scan image itself and a set of metadata. In this example, the metadata comprises the value of tissue radiodensity measured in Hounsfield Units (HU) of each pixel within the CT scan image, the resolution in cm²/pixels of the scan image (all of them embedded within the CT scan image), and an extra packet of data, inputted by the user of the computing device through a User Interface, which is related to the weight, height and age of the subject.

In step 403, further data corresponding to the subject is retrieved. Such data may be obtained from the CT scan image metadata or may be inputted externally from an interface. More precisely, the data relating to weight, height and age of the subject may be retrieved from the CT scan image metadata.

In step 404, the received CT scan image is segmented by a segmenting module of the computing device. The segmentation is performed by identifying a plurality of ROIs corresponding to predetermined types of tissues within the CT scan image. More precisely, the segmenting module identifies the part of the CT scan corresponding to the body of the subject, and other objects or parts depicted in the CT scan image which do not correspond to the body of the subject are discarded (i.e., area corresponding to the bed where the subject was lying when the CT scan is performed, or other objects or parts of the CT scan which may have accidentally showed up in the image, and are not part of the subject's body). Also, the segmenting module further identifies, within the identified part of the CT scan image corresponding to the body of the subject, the parts corresponding to Muscle, VAT and SAT tissues.

In step 405, a parameter corresponding to the area of each ROI (Region of Interest) is obtained. First, the area of the ROI corresponding to the body of the subject is calculated, using the resolution of the CT scan, in cm²/pixel. Furthermore, a parameter corresponding to the area of the other ROIs (Muscle, VAT and SAT tissues) is also obtained, by calculating the real area corresponding to these ROIs, using the resolution of the CT scan, in cm²/pixel.

The real areas of each identified ROI (either the total subject body, Muscle, VAT or SAT areas) are calculated by counting the number of pixels corresponding to the ROI for which the area is to be calculated, and multiplying said number of pixels by the pixel size in cm². The pixel size in cm² is obtained from the CT scan image metadata, for example, using a Pixel Spacing Attribute which may be included in the metadata. The pixel spacing attribute provides the physical distance in the patient between the center of each pixel, specified by a numeric pair (adjacent row spacing and adjacent column spacing, for example, in mm).

In step 406, a parameter corresponding to each of the areas of the ROIs corresponding to Muscle, VAT and SAT tissues is further calculated, in percentage respective to the ROI that corresponds to the entire body of the subject within the image. More precisely, each parameter is calculated by counting the number of pixels corresponding to the target ROI for which the area in percentage is to be calculated and dividing said number of pixels by the number of pixels corresponding to the ROI that corresponds to the entire body of the subject within the image. The value obtained is then multiplied by 100. This type of parameter may be a more objective metric than the area in cm² because it is a proportional value.

In step 407, a parameter corresponding to the average value of tissue radiodensity measured in Hounsfield Units (HU) is calculated for each ROI (body of the subject, Muscle, VAT and SAT), using the CT scan image metadata. More precisely, for each identified ROI, the computing device retrieves the values of tissue radiodensity of all the pixels corresponding to that ROI and calculates the average radiodensity of the specific ROI by calculating the arithmetic mean.

When assembling the training data sets to perform the training of neural network 200 according to the example of the present disclosure, it may be more computationally efficient that all input parameters are within the same numerical range in order to accelerate the algorithm convergence speed.

Therefore, since each input parameter of the neural network represents the quantification of a different feature of the subject, in step 408, each parameter is scaled using reasonable ranges (for example, it may be established that the weight of an adult human body may be between 45kg and 120kg). More precisely, a scaling is performed for each parameter, in order to scale all parameters so they take values inside or close to a desired numeric range, for example, 0 and 1. In the present example, all the parameters are scaled using the following reasonable values for each parameter:
Weight: 45 to 120 kg
Height: 1.4 to 2 m
Age: 0 to 100 years
Region of interest (ROI) (area): 250 to 1250 cm².
ROI (density): -100 to 100 HU
Muscle (%): 0 to 100
Muscle (density): -30 to 150 HU
visceral adipose tissue (VAT) (%): 0 to 100
VAT (density): -150 to -50 HU
subcutaneous adipose tissue (SAT) (%): 0 to 100
SAT (density): -190 to -30 HU
Intramuscular adipose tissue (IMAT) (%): 0 to 100
IMAT (density): -190 to -30 HU.

In examples of the present disclosure, the scaling of each parameter may be carried out using the following equation: scaled parameter = (unscaled parameter - minimum_reasonable_value) / (maximum_reasonable_value - minimum_reasonable_value)

Following this procedure, if the parameter value was included within the reasonable values range, its scaled value will be between 0 and 1 (both included). If the parameter value was higher than the maximum reasonable value, its scaled value will be higher than 1. If the parameter value was lower than the minimum reasonable value, its scaled value will be lower than 0.

Then, in step 409, a training data set is assembled, by coupling the scaled parameters based on the calculated parameters area in cm² of each ROI (body of the subject, Muscle, VAT and SAT), average radiodensity (in HU) of each ROI (body of the subject, Muscle, VAT and SAT), area the ROIs corresponding to Muscle, VAT and SAT tissues measured as a percentage (%) with respect to the area corresponding to the ROI that corresponds to the body of the subject within the image, and the retrieved parameters (weight, height and age of the subject), with the corresponding output parameter: the REE parameter received in step 401.

Then, in step 410, a supervised training is performed on the neural network, using the assembled training data set.

In step 411, once the neural network has been trained with a first training data set corresponding to a first subject, if the user wishes to keep training the neural network, the method may restart in step 401, wherein new IC test data or bioimpedanciometry test and a new CT scan image is used, corresponding to a new subject. Thus, the training method may be performed by the computing device in an iterative manner, using a plurality of training data sets from different subjects.

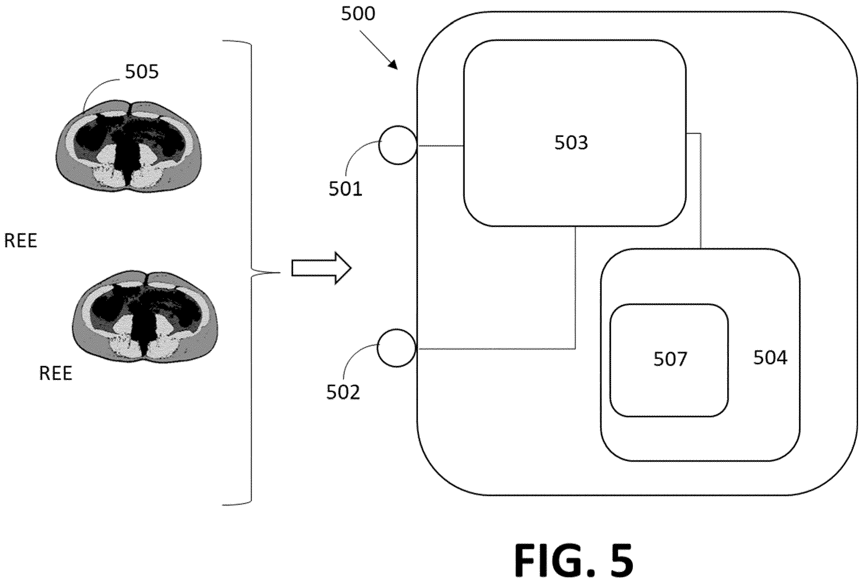

In examples, a solution according to the present disclosure and shown in figure 5 comprises a system 500 for providing a new estimated REE parameter 508 of a subject, the system comprising:
- an interface 501 to receive one or more computed tomography CT scan images 505; for illustration purposes, the image 505 is represented in figure 5 without non representative white contour around the region of the entire subject's body within the image;
- a second interface 502 to receive at least one ground-truth REE parameter for each corresponding CT scan image; the second interface 502 is represented as a different interface of the interface 501 but a unique interface may be used for the purposes of receiving at least one computed tomography CT scan image and at least one REE parameter for each corresponding CT scan image;
- a processor 503 in communication with the interfaces 501, 502; and
- non-transitory computer readable media 504 in communication with the processor that stores instruction code, which when executed by the processor, causes the processor to:
   - identify a ROI of each one or more computed tomography CT scan image;
   - calculate at least one parameter of each ROI the at least one parameter comprising a ROI radiodensity;
   - train a model 507 to estimate a REE parameter, using a supervised learning methodology, based on a training data set that comprises the at least one ground-truth REE parameter and the at least one parameter of each ROI, wherein the model receives as input the at least one ROI parameter and is told to infer or estimate the ground-truth REE parameter from the training data set.

In examples, once a trained model results from the supervised training, the model may be stored in non-volatile computer readable media.

In examples, the non-transitory computer readable media 504 further stores instruction code, which when executed by the processor 503, further causes the processor to:
- receive a new CT scan image of a new subject;
- process the new CT scan image to estimate a new REE parameter; and
- provide the new estimated REE parameter 508 via an output interface (not shown).

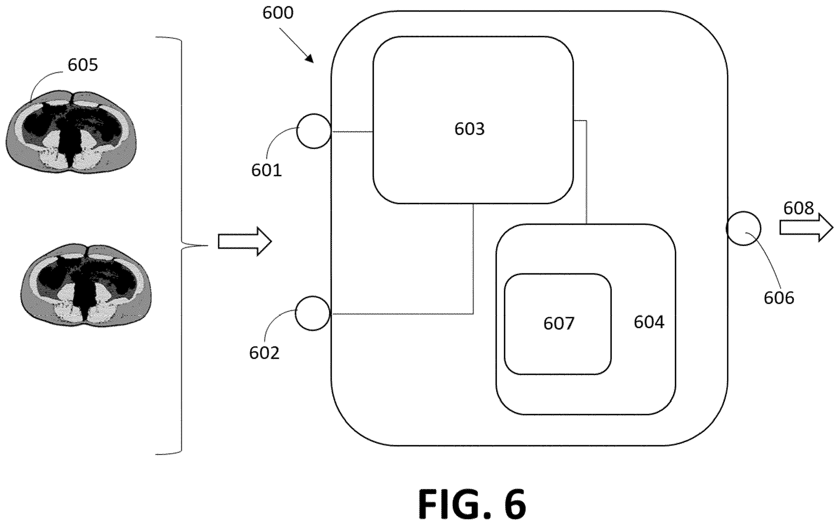

In examples, a solution according to the present disclosure comprises, as represented in figure 6, a system 600 for providing an estimated REE parameter 608 of a subject, the system comprising:
- one or more interfaces 601, 602 to receive one or more computed tomography CT scan images 605;
- an output interface 606 to provide the estimated REE parameter 608;
- a processor 603 in communication with the one or more interfaces 601, 602; and
- non-transitory computer readable media 604 in communication with the processor 603 that stores
   - a trained model 607 which has been trained using a supervised learning methodology as described in the present disclosure, based on a training data set that comprises at least one ground-truth REE parameter and at least one parameter of a ROI of one or more CT scan images, the at least one parameter comprising a ROI radiodensity;
   - instruction code, which when executed by the processor 603, causes the processor to:
      ▪ identify at least a ROI of a received CT scan images 605;
      ▪ calculate at least one parameter of the ROI;
      ▪ input the at least one parameter of the ROI to the trained model 607, obtaining an estimated REE parameter therefrom; and
      ▪ provide the estimated REE parameter 608 via the output interface 606.

In examples a solution according to the present disclosure comprises a method for providing a REE parameter of a subject, the method comprising:
- receiving a computed tomography CT scan image;
- identifying a ROI of the computed tomography CT scan image;
- calculate at least one parameter of the ROI;
- estimate the REE parameter using a model trained to estimate a REE parameter of a subject from at least one parameter of a ROI of at least one CT scan image; and
- providing the REE parameter of the subject.

In examples the above defined method for providing a REE parameter of a subject further includes training the model to estimate a new REE parameter, using a supervised learning methodology, based on a training data set that comprises the at least one training ROI parameter and the corresponding training REE parameter and, wherein the model receives as input the at least one training ROI parameter and is told to infer or estimate the training REE parameter from the training data set as output.

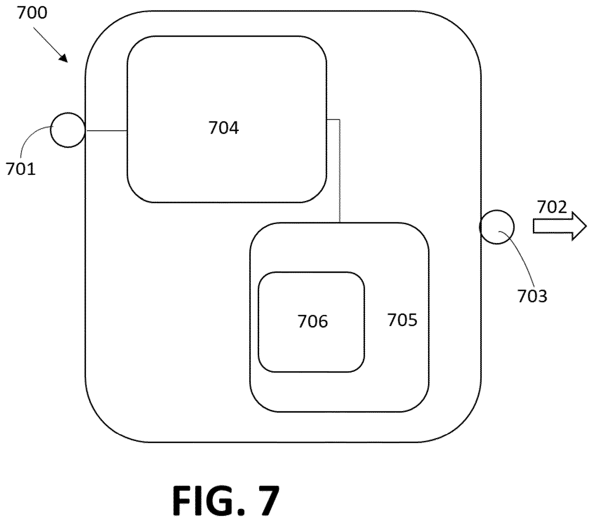

In some examples, a solution according to the present disclosure comprises, as represented in figure 7, a system 700 for providing an estimated REE parameter 702 of a subject, the system comprising:
- at least one interface 701 to receive one or more calculated parameters of a ROI of a CT scan image of a subject;
- an output interface 703 to provide the estimated REE parameter 702;
- a processor 704 in communication with the interface 701; and
- non-transitory computer readable media 705 in communication with the processor 704 that stores
   - a trained model 706 which has been trained using a supervised learning methodology as described in the present disclosure, based on a training data set that comprises at least one ground-truth REE parameter and at least one parameter of a ROI of one or more CT scan images;
   - instruction code, which when executed by the processor 704, causes the processor to:
      ▪ input the one or more calculated parameters of a ROI to the trained model 706, obtaining an estimated REE parameter 702 therefrom; and
      ▪ provide the estimated REE parameter 702 at the output interface 703.

Although only a number of examples have been disclosed herein, other alternatives, modifications, uses and/or equivalents thereof are possible. Furthermore, all possible combinations of the described examples are also covered. Thus, the scope of the present disclosure should not be limited by particular examples but should be determined only by a fair reading of the claims that follow. If reference signs related to drawings are placed in parentheses in a claim, they are solely for attempting to increase the intelligibility of the claim and shall not be construed as limiting the scope of the claim.

Further, although the examples described with reference to the drawings comprise computing apparatus/systems and processes performed in computing apparatus/systems, the invention also extends to computer programs, particularly computer programs on or in a carrier, adapted for putting the system into practice.

## Claims

1. A training method (400) of a computer-implemented machine learning model for obtaining a Resting Energy Expenditure, REE, parameter of a subject from one or more computed tomography, CT, scan images (300) of a subject, the method comprising the steps of:
- obtaining (401) one or more ground-truth REE parameters of one or more subjects;
- obtaining (402) the one or more CT scan images of the one or more subjects;
- identifying at least a region of interest, ROI, in each of the obtained CT scan images;
- calculating (405) a group of parameters of each ROI; wherein a group comprises one or more parameters of each ROI, wherein the one or more parameters comprise a radiodensity parameter for at least one of the identified ROIs, and wherein each group is associated with a ground-truth REE parameter; and
- performing (410) a supervised training of the computer-implemented machine learning, ML, model, using a training data set comprising one or more groups of parameters of each ROI and the associated one or more ground-truth REE parameters, and wherein the supervised training further comprises, for each group, setting an output parameter of the ML model corresponding to the associated ground-truth REE parameter.

2. A training method (400) according to claim 1 wherein
- identifying at least a region of interest, ROI, comprises segmenting (404) each of the obtained CT scan images, identifying thereby at least one predetermined type of tissue, organ, or any combination of them within the obtained image;
- and wherein calculating one or more parameters of each ROI comprises calculating at least one parameter related to an amount of the at least one identified ROI or related to a radiodensity of the ROI, from the segmented CT scan image.

3. A training method (400) according to claim 1 or 2 wherein the computer-implemented machine learning model is a regression model; or wherein the computer-implemented machine learning model is one of a neural network, or a model comprising assembled decision trees, or a neural network comprising at least one fully connected layer, or a combination of one or more neural networks and one or more decision trees.

4. A training method (400) according to any of claims 1 to 3 wherein the one or more parameters of each ROI are obtained by calculating an area or volume of an identified tissue, organ, or any combination of them from the CT scan image and a resolution of the CT scan image.

5. A training method (400) according to any of claims 1 to 4 wherein the one or more parameters of each ROI comprise a percentage of an identified ROI, relative to a tissue or an organ or to any combination of thereof, with regards to a different ROI relative to other tissues, other organs, or any combination of thereof, including an entire subject's body within the obtained CT scan image.

6. A training method (400) according to any of claims 1 to 5, wherein the one or more parameters further comprise an amount of a Muscle tissue, and/or of a visceral adipose tissue, VAT, and/or of a subcutaneous adipose tissue, SAT, and/or of an entire subject's body within the CT scan image.

7. A training method (400) according to any of claims 1 to 6, wherein the training data set further comprises at least one of demographic data, and/or anthropometric data and/or clinical data of the subject.

8. A training method (400) according to any of claims 1 to 7 wherein the steps of
- obtaining the one or more CT scan images of the one or more subjects;
- identifying at least a region of interest, ROI, in each of the obtained CT scan images; and
- calculating a group of parameters of each ROI
are performed by a first entity; and
the method further comprises receiving one or more parameters of each ROI by a second entity before performing a supervised training of the computer-implemented machine learning model by the second entity.

9. A method for inferring a Resting Energy Expenditure (REE) parameter of a subject, comprising inferring the Resting Energy Expenditure (REE) parameter using a computer-implemented machine learning model trained using the method of any of claims 1 to 8, wherein the inferring comprises inputting a calculated group of parameters into the machine learning model, and obtaining an REE parameter as the output of the model.

10. The method for inferring a REE parameter of claim 9 further comprising, before inferring the REE parameter:
- obtaining at least one CT scan image of the subject;
- identifying at least a ROI in the CT scan image; and
- calculating a group of parameters of each ROI, wherein the group of parameters comprise a radiodensity parameter for at least one of the identified ROls.

11. A data processing system (500), the system comprising a processor (503) configured to perform a supervised training of a computer-implemented machine learning model, using a training data set comprising one or more groups of parameters of respective ROI of CT scan images; wherein a group comprises one or more parameters of each ROI, and wherein each group is associated with a ground-truth REE parameter, wherein the group of parameters comprise a radiodensity parameter for at least one of the identified ROIs, and wherein the supervised training further comprises, for each group, setting an output parameter of the model corresponding to the associated ground-truth REE parameter.

12. The data processing system (500) of claim 11 further comprising:
- a first interface (502) to obtain the one or more ground-truth REE parameters of one or more subjects;
- a second interface (501) to obtain the one or more computed tomography, CT, scan images of the one or more subjects;
and wherein the processor (503) is further configured to:
- obtain the one or more CT scan images from the second interface;
- identify at least a region of interest, ROI, in each obtained CT scan image; and
- calculate at least one group of parameters of each ROI, wherein the group of parameters comprise a radiodensity parameter for at least one of the identified ROls.

13. The data processing system (500) of claim 11 or 12 wherein the processor is further configured, once a trained model results from the supervised training, to either:
- receive new one or more groups of parameters of a new ROI of a new CT scan image and to infer a new REE parameter using the trained model; or
- receive a new CT image obtained by the second interface; to identify a new region of interest, ROI, in the new obtained CT scan image; to calculate new one or more groups of parameters of the ROI, wherein the new one or more group of parameters comprise a radiodensity parameter for at least one of the identified ROIs; and to infer a new REE parameter using the trained model.

14. A data processing system (700) for providing an estimated REE parameter (702) of a subject, the system comprising
- at least one interface (701) to receive one or more calculated parameters of a ROI of a CT scan image of a subject;
- an output interface (703) to provide the estimated REE parameter (702);
- a processor (704) in communication with the interface (701); and
- non-transitory computer readable media (705) in communication with the processor (704) that stores:
- a trained model (706) which has been trained by implementing a method according to any one of claims 1 to 8, based on a training data set that comprises at least one ground-truth REE parameter and at least one parameter of a ROI of one or more CT scan images, wherein the parameter comprises a radiodensity parameter for at least one of the ROIs;
- instruction code, which when executed by the processor (704), causes the processor to:
- input the one or more calculated parameters of a ROI to the trained model (706), obtaining an estimated REE parameter (702) therefrom; and
- provide the estimated REE parameter (702) at the output interface (703).

15. A computer program product comprising program instructions for causing a computing system to perform a method (400) according to any one of claims 1 to 8.

## Patentansprüche

1. Ein Trainingsverfahren (400) eines computerimplementierten maschinellen Lernmodells zum Erhalten eines REE-Parameters (REE, *resting energy expenditure,* Ruheenergieausgabe) eines Patienten aus einem oder mehreren CT-Scanbildern (CT, *computed tomography*) (300) eines Patienten, wobei das Verfahren die folgenden Schritte umfasst:
- erhalten (401) eines oder mehrerer Ground-Truth-REE-Parameter eines oder mehrerer Patienten;
- erhalten (402) des einen oder der mehreren CT-Scanbilder des einen oder der mehreren Patienten;
- identifizieren mindestens einer Region von Interesse (ROI), in jedem der erhaltenen CT-Scanbilder;
- berechnen (405) einer Gruppe von Parametern jeder ROI; wobei eine Gruppe einen oder mehrere Parameter jeder ROI umfasst, wobei der eine oder die mehreren Parameter einen Radiodichteparameter für mindestens eine der identifizierten ROI umfassen, und wobei jede Gruppe einem Ground-Truth-REE-Parameter zugeordnet ist; und
- durchführen (410) eines überwachten Trainings des computerimplementierten ML-Modells (ML, *machine learning*) unter Verwendung eines Trainingsdatensatzes, der eine oder mehrere Gruppen von Parametern jeder ROI und den bzw. die zugeordneten einen bzw. mehreren Ground-Truth-REE-Parameter umfasst, und wobei das überwachte Training ferner für jede Gruppe das Einstellen eines Ausgabeparameters des ML-Modells umfasst, der dem zugeordneten Ground-Truth-REE-Parameter entspricht.

2. Ein Trainingsverfahren (400) nach Anspruch 1, wobei
- das Identifizieren mindestens einer Region von Interesse, ROI, das Segmentieren (404) jedes der erhaltenen CT-Scanbilder umfasst, wodurch mindestens eine vorbestimmte Art von Gewebe, von Organ oder einer beliebigen Kombination davon in dem erhaltenen Bild identifiziert wird;
- und wobei das Berechnen eines oder mehrerer Parameter jeder ROI das Berechnen mindestens eines Parameters in Bezug auf einen Betrag der mindestens einen identifizierten ROI oder in Bezug auf eine Radiodichte der ROI aus dem segmentierten CT-Scanbild umfasst.

3. Ein Trainingsverfahren (400) nach Anspruch 1 oder 2, wobei das computerimplementierte maschinelle Lernmodell ein Regressionsmodell ist; oder wobei das computerimplementierte maschinelle Lernmodell eines von einem neuronalen Netzwerk oder einem Modell ist, das zusammengesetzte Entscheidungsbäume umfasst, oder einem neuronalen Netzwerk, das mindestens eine vollständig verbundene Schicht umfasst, oder einer Kombination von einem oder mehreren neuronalen Netzwerken und einem oder mehreren Entscheidungsbäumen ist.

4. Ein Trainingsverfahren (400) nach einem der Ansprüche 1 bis 3, wobei der eine oder die mehreren Parameter jeder ROI durch Berechnen einer Fläche oder eines Volumens eines identifizierten Gewebes, Organs oder einer beliebigen Kombination davon aus dem CT-Scanbild und einer Auflösung des CT-Scanbilds erhalten werden.

5. Ein Trainingsverfahren (400) nach einem der Ansprüche 1 bis 4, wobei der eine oder die mehreren Parameter jeder ROI einen Prozentsatz einer identifizierten ROI in Bezug auf ein Gewebe oder ein Organ oder eine beliebige Kombination davon in Bezug auf eine andere ROI in Bezug auf andere Gewebe, andere Organe oder eine beliebige Kombination davon, einschließlich des gesamten Körpers eines Patienten in dem erhaltenen CT-Scanbild, umfassen.

6. Ein Trainingsverfahren (400) nach einem der Ansprüche 1 bis 5, wobei der eine oder die mehreren Parameter ferner eine Menge eines Muskelgewebes und/oder eines viszeralen Fettgewebes (VAT, *visceral adipose tissue*), und/oder eines subkutanen Fettgewebes (SAT, *subcutaneous adipose tissue*), und/oder des gesamten Körpers eines Patienten innerhalb des CT-Scanbilds umfassen.

7. Ein Trainingsverfahren (400) nach einem der Ansprüche 1 bis 6, wobei der Trainingsdatensatz ferner mindestens eines von demografischen Daten und/oder anthropometrischen Daten und/oder klinischen Daten des Patienten umfasst.

8. Ein Trainingsverfahren (400) nach einem der Ansprüche 1 bis 7, wobei die folgenden Schritte
- erhalten des einen oder der mehreren CT-Scanbilder des einen oder der mehreren Patienten;
- identifizieren mindestens einer Region von Interesse, ROI, in jedem der erhaltenen CT-Scanbilder; und
- berechnen einer Gruppe von Parametern jeder ROI
von einer ersten Einheit durchgeführt werden; und
das Verfahren ferner das Empfangen eines oder mehrerer Parameter jeder ROI durch eine zweite Einheit vor dem Durchführen eines überwachten Trainings des computerimplementierten maschinellen Lernmodells durch die zweite Einheit umfasst.

9. Ein Verfahren zum Ableiten eines REE-Parameters eines Patienten, umfassend das Ableiten des REE-Parameters unter Verwendung eines computerimplementierten maschinellen Lernmodells, das unter Verwendung des Verfahrens von einem der Ansprüche 1 bis 8 trainiert wurde, wobei das Ableiten das Eingeben einer berechneten Gruppe von Parametern in das maschinelle Lernmodell und das Erhalten eines REE-Parameters als die Ausgabe des Modells umfasst.

10. Das Verfahren zum Ableiten eines REE-Parameters von Anspruch 9, ferner umfassend, vor dem Ableiten des REE-Parameters:
- erhalten mindestens eines CT-Scanbildes des Patienten;
- identifizieren mindestens einer ROI in dem CT-Scanbild; und
- berechnen einer Gruppe von Parametern jeder ROI, wobei die Gruppe von Parametern einen Radiodichteparameter für mindestens eine der identifizierten ROI umfasst.

11. Ein Datenverarbeitungssystem (500), wobei das System einen Prozessor (503) umfasst, der konfiguriert ist, um ein überwachtes Training eines computerimplementierten maschinellen Lernmodells unter Verwendung eines Trainingsdatensatzes durchzuführen, der eine oder mehrere Gruppen von Parametern von jeweiligen ROI von CT-Scanbildern umfasst; wobei eine Gruppe einen oder mehrere Parameter von jeder ROI umfasst, und wobei jede Gruppe einem Ground-Truth-REE-Parameter zugeordnet ist, wobei die Gruppe von Parametern einen Radiodichteparameter für mindestens eine der identifizierten ROI umfasst, und wobei das überwachte Training ferner für jede Gruppe das Einstellen eines Ausgabeparameters des Modells umfasst, der dem zugeordneten Ground-Truth-REE-Parameter entspricht.

12. Das Datenverarbeitungssystem (500) von Anspruch 11, ferner umfassend:
- eine erste Schnittstelle (502), um den einen oder die mehreren Ground-Truth-REE-Parameter eines oder mehrerer Patienten zu erhalten;
- eine zweite Schnittstelle (501), um das eine oder die mehreren CT-Scanbilder des einen oder der mehreren Patienten zu erhalten;
und wobei der Prozessor (503) ferner dazu konfiguriert ist:
- das eine oder die mehreren CT-Scanbilder von der zweiten Schnittstelle zu erhalten;
- mindestens eine Region von Interesse, ROI, in jedem erhaltenen CT-Scanbild zu identifizieren; und
- mindestens eine Gruppe von Parametern jeder ROI zu berechnen, wobei die Gruppe von Parametern einen Radiodichteparameter für mindestens eine der identifizierten ROI umfasst.

13. Das Datenverarbeitungssystem (500) von Anspruch 11 oder 12, wobei der Prozessor ferner dazu konfiguriert ist, sobald ein trainiertes Modell aus dem überwachten Training entsteht, entweder:
- eine neue oder mehrere neue Gruppen von Parametern einer neuen ROI eines neuen CT-Scanbilds zu empfangen und einen neuen REE-Parameter unter Verwendung des trainierten Modells abzuleiten; oder
- ein neues CT-Bild zu empfangen, das durch die zweite Schnittstelle erhalten wurde; eine neue Region von Interesse, ROI, in dem neuen erhaltenen CT-Scanbild zu identifizieren; eine neue oder mehrere neue Gruppen von Parametern der ROI zu berechnen, wobei die neue oder die mehreren neuen Gruppen von Parametern einen Radiodichteparameter für mindestens eine der identifizierten ROI umfassen; und einen neuen REE-Parameter unter Verwendung des trainierten Modells abzuleiten.

14. Ein Datenverarbeitungssystem (700) zum Bereitstellen eines geschätzten REE-Parameters (702) eines Patienten, wobei das System Folgendes umfasst:
- mindestens eine Schnittstelle (701) zum Empfangen eines oder mehrerer berechneter Parameter einer ROI eines CT-Scanbilds eines Patienten;
- eine Ausgabeschnittstelle (703), um den geschätzten REE-Parameter (702) bereitzustellen;
- einen Prozessor (704) in Kommunikation mit der Schnittstelle (701); und
- ein nichtflüchtiges computerlesbares Medium (705) in Kommunikation mit dem Prozessor (704), das Folgendes speichert:
- ein trainiertes Modell (706), welches durch Implementieren eines Verfahrens nach einem der Ansprüche 1 bis 8 trainiert wurde, basierend auf einem Trainingsdatensatz, der mindestens einen Ground-Truth-REE-Parameter und mindestens einen Parameter einer ROI von einem oder mehreren CT-Scanbildern umfasst, wobei der Parameter einen Radiodichteparameter für mindestens eine der ROI umfasst;
- Anweisungscode, welcher bei Ausführung durch den Prozessor (704) den Prozessor zu Folgendem veranlasst:
- eingeben des einen oder der mehreren berechneten Parameter einer ROI in das trainierte Modell (706), erhalten eines geschätzten REE-Parameters (702) daraus; und
- den geschätzten REE-Parameter (702) an der Ausgabeschnittstelle (703) bereitzustellen.

15. Ein Computerprogrammprodukt umfassend Programmanweisungen zum Veranlassen eines Computersystems, ein Verfahren (400) nach einem der Ansprüche 1 bis 8 durchzuführen.

## Revendications

1. Un procédé de formation (400) d'un modèle d'apprentissage automatique mis en œuvre par ordinateur pour obtenir un paramètre de REE (*Resting Energy Expenditure,* dépense d'énergie au repos) d'un sujet à partir d'une ou de plusieurs images de tomodensitométrie, ou CT, (*computed tomography*) (300) d'un sujet, le procédé comprenant les étapes consistant à :
- obtenir (401) un ou plusieurs paramètres de REE *ground truth* (« vérité de terrain ») d'un ou de plusieurs sujets ;
- obtenir (402) la une ou les plusieurs images de tomodensitométrie de l'un ou des plusieurs sujets ;
- identifier au moins une région d'intérêt, ROI, dans chacune des images de tomodensitométrie obtenues ;
- calculer (405) un groupe de paramètres de chaque ROI ; dans lequel un groupe comprend un ou plusieurs paramètres de chaque ROI, dans lequel le un ou les plusieurs paramètres comprennent un paramètre de radiodensité pour au moins l'une des ROI identifiées, et dans lequel chaque groupe est associé à un paramètre de REE *ground truth* ; et
- réaliser (410) une formation supervisée du modèle d'apprentissage automatique, ML (*machine learning*), mis en œuvre par ordinateur, à l'aide d'un ensemble de données de formation comprenant un ou plusieurs groupes de paramètres de chaque ROI et le ou les paramètres REE *ground truth* associés, et dans lequel la formation supervisée comprend en outre, pour chaque groupe, établir un paramètre de sortie du modèle de ML correspondant au paramètre de REE *ground truth* associé.

2. Un procédé de formation (400) selon la revendication 1, dans lequel
- identifier au moins une région d'intérêt, ROI, comprend la segmentation (404) de chacune des images de tomodensitométrie obtenues, identifiant ainsi au moins un type prédéterminé de tissu, d'organe ou toute combinaison de ceux-ci dans l'image obtenue ;
- et dans lequel le calcul d'un ou de plusieurs paramètres de chaque ROI comprend le calcul d'au moins un paramètre lié à une quantité de la au moins une ROI identifiée ou lié à une radiodensité de la ROI, à partir de l'image de tomodensitométrie segmentée.

3. Un procédé de formation (400) selon la revendication 1 ou 2, dans lequel le modèle d'apprentissage automatique mis en œuvre par ordinateur est un modèle de régression ; ou dans lequel le modèle d'apprentissage automatique mis en œuvre par ordinateur est l'un d'un réseau neuronal, ou d'un modèle comprenant des arbres de décision assemblés, ou d'un réseau neuronal comprenant au moins une couche entièrement connectée, ou d'une combinaison d'un ou plusieurs réseaux neuronaux et d'un ou plusieurs arbres de décision.

4. Un procédé de formation (400) selon l'une quelconque des revendications 1 à 3, dans lequel le un ou les plusieurs paramètres de chaque ROI sont obtenus en calculant une superficie ou un volume d'un tissu identifié, d'un organe identifié ou d'une combinaison quelconque de ceux-ci, à partir de l'image de tomodensitométrie et d'une résolution de l'image de tomodensitométrie.

5. Un procédé de formation (400) selon l'une quelconque des revendications 1 à 4, dans lequel l'un ou les plusieurs paramètres de chaque ROI comprennent un pourcentage d'une ROI identifiée, par rapport à un tissu ou un organe ou à toute combinaison de ceux-ci, par rapport à une ROI différente par rapport à d'autres tissus, d'autres organes ou une combinaison quelconque de ceux-ci, y compris le corps entier d'un sujet dans l'image de tomodensitométrie obtenue.

6. Un procédé de formation (400) selon l'une quelconque des revendications 1 à 5, dans lequel le un ou les plusieurs paramètres comprennent en outre une quantité d'un tissu musculaire, et/ou d'un tissu adipeux viscéral, TAV, et/ou d'un tissu adipeux sous-cutané, TASC, et/ou du corps entier d'un sujet dans l'image de tomodensitométrie.

7. Un procédé de formation (400) selon l'une quelconque des revendications 1 à 6, dans lequel l'ensemble de données de formation comprend en outre au moins l'une parmi de données démographiques et/ou de données anthropométriques et/ou de données cliniques du sujet.

8. Un procédé de formation (400) selon l'une quelconque des revendications 1 à 7, dans lequel les étapes consistant à
- obtenir la une ou les plusieurs images de tomodensitométrie de l'un ou des plusieurs sujets ;
- identifier au moins une région d'intérêt, ROI, dans chacune des images de tomodensitométrie obtenues ; et
- calculer un groupe de paramètres de chaque ROI
sont effectuées par une première entité ; et
le procédé comprend en outre la réception d'un ou de plusieurs paramètres de chaque ROI par une seconde entité avant d'effectuer une formation supervisée du modèle d'apprentissage automatique mis en œuvre par ordinateur par la seconde entité.

9. Un procédé pour inférer un paramètre de dépense d'énergie au repos (REE) d'un sujet, comprenant l'inférence du paramètre de dépense d'énergie au repos (REE) en utilisant un modèle d'apprentissage automatique mis en œuvre par ordinateur formé en utilisant le procédé de l'une quelconque des revendications 1 à 8, dans lequel l'inférence comprend l'entrée d'un groupe calculé de paramètres dans le modèle d'apprentissage automatique, et l'obtention d'un paramètre de REE en tant que sortie du modèle.

10. Le procédé pour inférer un paramètre de REE de la revendication 9, comprenant en outre, avant d'inférer le paramètre de REE :
- obtenir au moins une image de tomodensitométrie du sujet ;
- identifier au moins une ROI dans l'image de tomodensitométrie ; et
- calculer un groupe de paramètres de chaque ROI, dans lequel le groupe de paramètres comprend un paramètre de radiodensité pour au moins l'une des ROI identifiées.

11. Un système de traitement de données (500), le système comprenant un processeur (503) configuré pour effectuer une formation supervisée d'un modèle d'apprentissage automatique mis en œuvre par ordinateur, en utilisant un ensemble de données de formation comprenant un ou plusieurs groupes de paramètres de ROI respectifs d'images de tomodensitométrie ; dans lequel un groupe comprend un ou plusieurs paramètres de chaque ROI, et dans lequel chaque groupe est associé à un paramètre de REE *ground truth,* dans lequel le groupe de paramètres comprend un paramètre de radiodensité pour au moins l'une des ROI identifiées, et dans lequel la formation supervisée comprend en outre, pour chaque groupe, établir un paramètre de sortie du modèle correspondant au paramètre de REE *ground truth* associé.

12. Le système de traitement de données (500) de la revendication 11, comprenant en outre :
- une première interface (502) pour obtenir l'un ou les plusieurs paramètres REE *ground truth* d'un ou de plusieurs sujets ;
- une seconde interface (501) pour obtenir la une ou les plusieurs images de tomodensitométrie de l'un ou des plusieurs sujets ;
et dans lequel le processeur (503) est en outre configuré pour :
- obtenir la une ou les plusieurs images de tomodensitométrie à partir de la seconde interface ;
- identifier au moins une région d'intérêt, ROI, dans chaque image de tomodensitométrie obtenue ; et
- calculer au moins un groupe de paramètres de chaque ROI, dans lequel le groupe de paramètres comprend un paramètre de radiodensité pour au moins l'une des ROI identifiées.

13. Le système de traitement de données (500) de la revendication 11 ou 12, dans lequel le processeur est en outre configuré pour, une fois qu'un modèle formé résulte de la formation supervisée :
- recevoir un nouveau ou plusieurs nouveaux groupes de paramètres d'une nouvelle ROI d'une nouvelle image de tomodensitométrie et en inférer un nouveau paramètre de REE à l'aide du modèle formé ; ou
- recevoir une nouvelle image de tomodensitométrie obtenue par la seconde interface ; identifier une nouvelle région d'intérêt, ROI , dans la nouvelle image de tomodensitométrie obtenue ; calculer un nouveau ou plusieurs nouveaux groupes de paramètres de la ROI, dans lequel le nouveau ou les nouveaux groupes de paramètres comprennent un paramètre de radiodensité pour au moins l'une des ROI identifiées ; et inférer un nouveau paramètre de REE en utilisant le modèle formé.

14. Un système de traitement de données (700) pour fournir un paramètre de REE estimé (702) d'un sujet, le système comprenant
- au moins une interface (701) pour recevoir un ou plusieurs paramètres calculés d'une ROI d'une image de tomodensitométrie d'un sujet ;
- une interface de sortie (703) pour fournir le paramètre de REE estimé (702) ;
- un processeur (704) en communication avec l'interface (701) ; et
- un support lisible par ordinateur non transitoire (705) en communication avec le processeur (704) qui stocke :
- un modèle formé (706) qui a été formé par la mise en œuvre d'un procédé selon l'une quelconque des revendications 1 à 8, sur la base d'un ensemble de données de formation qui comprend au moins un paramètre de REE *ground truth* et au moins un paramètre d'une ROI d'une ou plusieurs images de tomodensitométrie, dans lequel le paramètre comprend un paramètre de radiodensité pour au moins l'une des ROI ;
- du code d'instruction, qui, lorsqu'il est exécuté par le processeur (704), amène le processeur à :
- entrer le un ou les plusieurs paramètres calculés d'une ROI dans le modèle formé (706), en obtenant un paramètre de REE estimé (702) à partir de celui-ci ; et
- fournir le paramètre de REE estimé (702) au niveau de l'interface de sortie (703).

15. Un produit de programme informatique comprenant des instructions de programme pour amener un système informatique à exécuter un procédé (400) selon l'une quelconque des revendications 1 à 8.
